# EUROPÄISCHE PATENTSCHRIFT

(11) **EP 0 318 649 B1**
(45) Veröffentlichungstag und Bekanntmachung des Hinweises auf die Patenterteilung: **03.01.1996**
(21) Anmeldenummer: 88114589.0
(22) Anmeldetag: 07.09.1988
(51) Int. Cl.: G03F 7/004, G03C 1/72, C07C 43/225, C07C 43/313, C07C 43/315, C07C 69/16, C07C 69/78, C07C 69/96, C07F 7/18

(54) **Positiv arbeitendes strahlungsempfindliches Gemisch und daraus hergestelltes strahlungsempfindliches Aufzeichnungsmaterial für hochenergetische Strahlung**
Positive-working radiation-sensitive composistion and radiation-sensitive recording material produced therefrom for high-energy radiation
Composistion sensible aux rayonnements à effet positif et matière d'enregistrement sensible à rayonnements et produits pour rayonnements de haute énergie

(30) Priorität: 13.09.1987 DE 3730784; 25.06.1988 DE 3821585
(43) Veröffentlichungstag der Anmeldung: 07.06.1989
(73) Patentinhaber: HOECHST AKTIENGESELLSCHAFT, D-65926 Frankfurt am Main (DE)
(72) Erfinder: Dössel, Karl-Friedrich, Dr., D-6200 Wiesbaden (DE); Dammel, Ralph, Dr., D-6500 Mainz-Bretzenheim (DE); Lingnau, Jürgen, Dr., D-6500 Mainz-Laubenheim (DE)

(56) Entgegenhaltungen:
- EP-A- 0 059 250
- EP-A- 0 078 981
- EP-A- 0 111 274
- DE-A- 2 342 068
- DE-A- 2 610 842
- DE-A- 2 742 631
- US-A- 3 515 552

## Beschreibung

Positiv arbeitendes strahlungsempfindliches Gemisch und daraus hergestelltes strahlungsempfindliches Aufzeichnungsmaterial für hochenergetische Strahlung

Die Erfindung betrifft ein positiv arbeitendes strahlungsempfindliches Gemisch, enthaltend eine Verbindung, die unter Einwirkung von hochenergetischer Strahlung eine Säure bildet sowie eine Verbindung, die durch Säure spaltbar ist.

Bei der klassischen UV-Lithographie ist die Grenze der Auflösung durch die Wellenlänge der verwendeten Strahlung vorgegeben. Die stetige Verkleinerung der Dimensionen bei der Chip-Herstellung erfordert daher im Submikronbereich neue Lithographietechniken, wobei wegen ihrer extrem kurzen Wellenlänge Elektronen- oder Röntgenstrahlung eingesetzt werden. Dabei hat sich gezeigt, daß Resistmaterialien, die als Elektronenstrahlresist geeignet sind, auch als Röntgenstrahlresist eingesetzt werden können und umgekehrt.

Bekannte Resistmaterialien für diese Anwendung sind Acrylate und Methacrylate (G.M. Taylor, Solid State Technology, 124 (1984)). Bei diesen Materialien zeigte sich, daß Empfindlichkeit und Strukturauflösung meist gegenläufige Eigenschaften sind. Sollen höhere Empfindlichkeiten erreicht werden können, werden meist Halogene in den Resist eingebaut. Dabei wird in positiv arbeitenden Resists meist Fluor und Chlor, in negativ arbeitenden Resists dagegen neben Chlor eher Brom und Iod eingesetzt (T. Yamaoka et al., Phot. Sci. Eng. 23, 196 (1979)).

Im allgemeinen zeigen negativ arbeitende Resists eine höhere Empfindlichkeit als positiv arbeitende, können dagegen aber nicht - wie oben ausgeführt - gleichzeitig eine hohe Auflösung im Submikronbereich aufweisen. Andererseits erreichen positiv arbeitende Resists auf Methacrylatbasis eine hohe Auflösung, sind aber mit Ausnahme der Resists auf Polymethacrylnitril-Basis nicht gegen die zur Halbleiterstrukturierung verwendeten Plasmaätzprozesse stabil. Die Methacrylate wiederum sind aber nicht genügend empfindlich.

Die Polymeren mit der bisher höchsten bekannten Strahlungsempfindlichkeit für Elektronen- bzw. Röntgenstrahlen sind Polyalkensulfone, insbesondere das Polybuten-1-sulfon. Der Nachteil dieser Verbindungsklasse liegt aber in ihrer weniger guten Resistenz gegenüber Plasmaätzprozessen; sie sind daher zwar für die Maskenherstellung geeignet, nicht aber für die Halbleiterfertigung mit einer Maske aus diesem Material. Es wurde daher vorgeschlagen, Polyalkensulfone mit den bekanntermaßen plasmaätzresistenten Novolakharzen zu kombinieren (M.J. Bowden et al., J. Electrochem. Soc. 128, 1304 (1981); US-A-4 289 845). Es zeigte sich aber eine starke Unverträglichkeit der beiden Polymeren miteinander, wodurch die Auflösung beeinträchtigt wird. Auch der Versuch, die Verträglichkeit durch Beimischung weiterer Komponenten zu verbessern, mußte mit einem Verlust an Empfindlichkeit erkauft werden (US-A-4 398 001).

Photokatalytische Systeme werden für die Anwendung mit Elektronen- und Röntgenstrahlung in der DE-A-27 18 254 und DE-A-29 28 636 beschrieben. In diesen positiv arbeitenden Systemen werden als Verbindungen, die unter Einwirkung von aktinischer Strahlung eine Säure bilden, chlorhaltige Verbindungen, insbesondere des substituierten Triazintyps, eingesetzt. Bei der Strukturierung dieser Materialien mit Elektronen- oder Röntgenstrahlung zeigte sich aber, daß die Flanken der Resiststrukturen nach dem Entwickeln stark negativ unterhöhlt sind (Flankenwinkel um 60° nach einer Aufnahme mit einem Rasterelektronenmikroskop (REM)) und als Folge davon Strukturen unter ca. 2 µm nicht mehr aufgelöst bzw. abgebildet werden können oder aber zum Teil sehr stark ausgefranst sind.

In der DE-A-29 28 636 wird, wie auch in der DE-A26 10 842, als chlorhaltige Verbindung u.a. 2,3,4,5Tetrachloranilin genannt. Darüber hinaus werden in der DE-A-26 10 842 auch Verbindungen offenbart, die aliphatisch gebundenes Brom enthalten sowie eine, die Brom aromatisch gebunden trägt: 2,2',4,4',6,6'-Hexabromdiphenylamin. Es zeigte sich aber, daß mit der aromatischen Chlorverbindung keine genügend hohe Auflösung erzielt werden kann; mit der genannten Verbindung, die aromatisch gebundenes Brom enthält, traten dagegen unerwünschte Bildschleier auf.

Es bestand daher die Aufgabe, ein positiv arbeitendes strahlungsempfindliches Gemisch bereitzustellen, das für die Anwendung als Elektronen- bzw. Röntgenresist geeignet ist, eine höhere Empfindlichkeit und eine verbesserte Auflösung aufweist, keine Bildschleier nach dem Entwickeln mit einem wäßrig alkalischen Entwickler zeigt und eine hinreichende Resistenz gegenüber dem Plasmaätzen besitzt.

Gelöst wird die Aufgabe durch Bereitstellen eines positiv arbeitenden strahlungsempfindlichen Gemisches, enthaltend eine Verbindung, die unter Einwirkung von hochenergetischer Strahlung eine Säure bildet, sowie eine Verbindung, die durch Säure spaltbar ist, dadurch gekennzeichnet, daß die Verbindung, die eine Säure bildet, aromatisch gebundenes Chlor oder Brom enthält und einen pKₐ-Wert von kleiner als 12 aufweist oder ein Derivat einer Verbindung mit einem derartigen pKₐ-Wert ist.

Es war völlig überraschend, daß Verbindungen bzw. Starter, die aromatisch gebundenes Chlor oder Brom enthalten und einen pKₐ-Wert von kleiner als 12 aufweisen, bzw. deren Derivate diese vorteilhaften Eigenschaften besitzen, nachdem die im Stand der Technik (DE-A-26 10 842) geoffenbarten, aromatisch gebundenes Halogen, insbesondere Brom enthaltenden Verbindungen, sich als wenig geeignet erwiesen hatten.

Von den säurebildenden Startern, die aromatisch gebundenes Chlor oder Brom enthalten, werden solche bevorzugt, die einen pKₐ-Wert im Bereich von 6 bis 10 aufweisen bzw. Derivate dieser Verbindungen.

Der pKₐ-Wert von chemischen Verbindungen läßt sich nach herkömmlichen Methoden bestimmen, es ist aber auch eine theoretische Berechnung mit dem Programm "CAMEO" u.ä. möglich.

Darüber hinaus zeigen die als Starter eingesetzten Verbindungen im Gegensatz zu den bekannten Startern eine geringe Absorption im Spektralbereich oberhalb etwa 300 nm und ermöglichen somit das Arbeiten auch unter konventioneller Laborbeleuchtung. Gegenüber der in der EP-A-0 111 274 genannten Kombination von bromiertem Polyp-vinylphenol mit Starterverbindungen, die im wesentlichen im Bereich des nahen UV-Lichts oberhalb 300 nm absorbieren, ergeben sich neben diesen Vorteilen auch vereinfachte Formulierungen, geringere Probleme mit Verträglichkeiten und schließlich durch die Abmischung mit Novolaken als Bindemitteln eine gezielte Steuerungsmöglichkeit der Entwicklungsgeschwindigkeit und damit auch der Strahlungsempfindlichkeit.

Als säurebildende Starter kommen solche in Betracht, die mindestens ein in dem genannten pKₐ-Bereich als Proton abspaltbares H-Atom aufweisen. Hierzu gehören insbesondere Verbindungen mit Carboxylgruppen, phenolischen OH-Gruppen, SH-Gruppen oder entsprechend aktivierten Säureamidgruppen. Es ist jedoch nicht erforderlich, daß diese sauren Gruppen in freier Form vorliegen. Sie können auch durch geeignete, unter den Bedingungen der Verarbeitung abspaltbare Schutzgruppen, z.B. Acetal-, Ester-und bestimmte Ethergruppen, wie sie in den weiter unten aufgeführten säurespaltbaren Verbindungen enthalten sind, geschützt sein. Unter bestimmten Voraussetzungen sind auch solche Verbindungen als Starter geeignet, in denen die genannten sauren Gruppen irreversibel substituiert sind. Wenn derartige Verbindungen in wäßrig entwickelbaren Gemischen eingesetzt werden sollen, ist es jedoch erforderlich, daß diese Gemische wesentliche Mengenanteile an alkalilöslichen Bindemitteln enthalten.

Besonders bevorzugt sind säurebildende Starter der allgemeinen Formel I 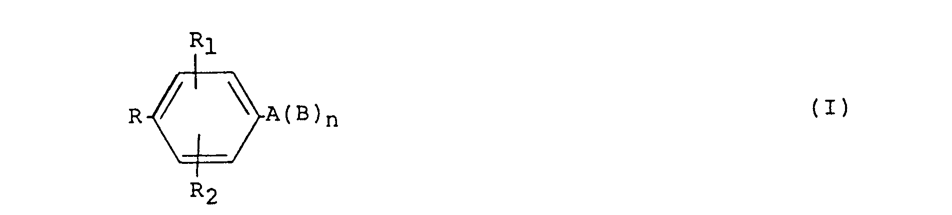 mit mindestens einem aromatisch gebundenen Chlor- oder Bromatom, worin
- R: Carboxyl, OR' oder SR',
- R₁ und R₂: gleich oder verschieden sind und Wasserstoff, Chlor, Brom, Alkyl, ggf. substituiert durch Aryl-, Alkoxy-, Aryloxy-, Hydroxygruppen oder Fluoratome; Aryl, ggf. substituiert durch Alkoxy-, Aryloxy-, Hydroxygruppen oder Halogenatome,
- R': Wasserstoff, Alkyl, ggf. substituiert durch Aryl-, Alkoxy-, Aryloxy-, Hydroxygruppen oder Fluoratome; Aryl, ggf. substituiert durch Alkoxy-, Aryloxy-, Hydroxygruppen oder Halogenatome; Acyl, Alkylsulfonyl, Arylsulfonyl, Alkoxycarbonyl, Triorganosilyl, Triorganostannyl oder eine Alkylen-, Arylen-, Bisacyl-, Sulfonyl-, Alkylendisulfonyl-, Arylendisulfonyl-, Diorganosilyl- oder Diorganostannylgruppe ist, deren zweite Valenz an das O-Atom einer weiteren Einheit der Formel I gebunden ist, wobei die Alkylen- und Arylengruppen entsprechend substituiert sein können wie die Alkyl-und Arylreste,
- und n: 0 bis 3 bedeutet, wobei
für n = 0:
A Wasserstoff, Chlor, Brom, Alkyl, ggf. substituiert durch Alkoxy-, Aryloxy-, Hydroxy-, Arylreste oder Fluoratome; Aryl, ggf. substituiert durch Alkoxy-, Aryloxy-, Hydroxy-, Carboxylreste oder Halogenatome, für n = 1:
A eine Einfachbindung, -O-, -S-, -SO₂-, -NH-, -NR₃-, Alkylen, Perfluoralkylen, für n = 2:
A 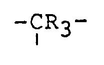 oder 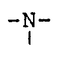 und für n = 3:
A 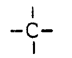 bedeutet, sowie
B 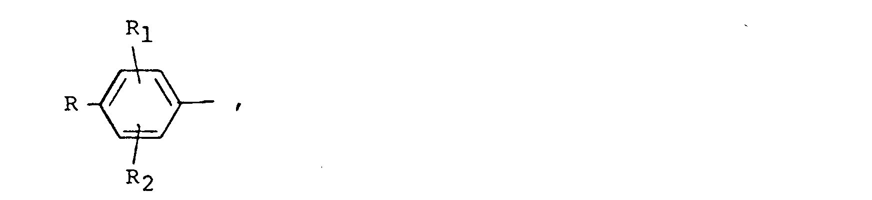 Carboxyl, substituiertes Carbonyl, insbesondere Alkyl- oder Arylcarbonyl, Carboxyalkyl sowie substituiertes Sulfonylimidocarbonyl und
R₃ Alkyl, insbesondere (C₁-C₃)Alkyl, oder Aryl, insbesondere Phenyl,
bedeutet.

Ganz besonders bevorzugt ist, wenn
- R₁ und R₂: gleich sind und insbesondere Brom bedeuten, wobei sie jeweils in ortho-Position zu R stehen, sowie
- R: eine freie oder durch eine mittels Säure abspaltbare Gruppe geschützte Hydroxygruppe ist.

Dabei wird ebenso besonders bevorzugt, wenn
- A: (für n=1 und B gleich 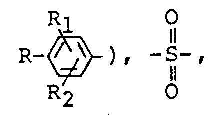 Propylen oder Perfluorpropylen, wobei die beiden zuletzt genannten Substituenten vorzugsweise jeweils am gleichen C-Atom durch 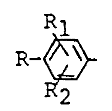 substituiert sind, oder
- A: (für n=1 und B gleich Alkylcarbonyl, insbesondere Methylcarbonyl, Carboxyalkyl, insbesondere Carboxymethyl, substituiertes Sulfonylimidocarbonyl, insbesondere p-Toluolsulfonylimidocarbonyl) -O-, -NH- oder -NR₃- oder
- A: (für n=0) Hydroxyl, Carboxyl oder Alkyl, welches an jedem zweiten C-Atom durch Phenyl, ggf. ein- oder mehrfach Brom enthaltend, substituiert ist,
bedeutet.

Insbesondere bevorzugt sind Verbindungen, in denen für n=0 und A gleich substituiertes Carbonyl:
- R: Hydroxyl und
- R₁ und R₂: jeweils in ortho-Position zu R stehend Brom bedeuten, oder
für n=0 und A gleich ggf. substituiertes Alkyl:
- R: Hydroxyl und
- R₁ und R₂: jeweils in ortho-Position zu R stehend Wasserstoff oder Brom bedeuten.

Der Gehalt an säurebildenden Startern in dem erfindungsgemäßen strahlungsempfindlichen Gemisch liegt im allgemeinen bei 2 bis 50 Gew.-%, vorzugsweise bei 4 bis 25 Gew.-%, jeweils bezogen auf das Gesamtgewicht der Schicht.

Als säurespaltbares Material in dem erfindungsgemäßen strahlungsempfindlichen Gemisch haben sich vor allem folgende Verbindungsklassen bewährt:
a) solche mit mindestens einer Orthocarbonsäureester-und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können,
b) Oligomer- oder Polymerverbindungen mit wiederkehrenden Acetal- und/oder Ketalgruppierungen in der Hauptkette,
c) Verbindungen mit mindestens einer Enolether-, oder N-Acyliminocarbonatgruppierung,
d) cyclische Acetale oder Ketale von β-Ketoestern oder -amiden,
e) Verbindungen mit Silylethergruppierungen,
f) Verbindungen mit Silyl-enolethergruppierungen,
g) Monoacetale bzw. Monoketale, deren Aldehyd- bzw. Ketonkomponente eine Löslichkeit im Entwickler zwischen 0,1 und 100 g/1 aufweisen,
h) Ether auf Basis tertiärer Alkohole und
i) Carbonsäureester und Carbonate tertiärer, allylischer oder benzylischer Alkohole

Durch Säure spaltbare Verbindungen des Typs a) als Komponenten strahlungsempfindlicher Gemische sind in der EP-A-0 022 571 und der DE-A-26 10 842 ausführlich beschrieben; Gemische, die Verbindungen des Typs b) enthalten, sind in der DE-C-23 06 248 und der DE-C27 18 254 dargestellt; Verbindungen des Typs c) werden in den EP-A-0 006 626 und 0 006 627 beschrieben; Verbindungen des Typs d) werden in der EP-A 0 202 196 und Verbindungen, die dem Typ e) zuzurechnen sind, in den DE-A3 544 165 und DE-A-3 601 264 vorgestellt; Verbindungen des Typs f) findet man in der gleichzeitig eingereichten EP-A-0 324 059, während Verbindungen vom Typ g) in den ebenfalls gleichzeitig eingereichten EP-A-0 312 751 und EP-A-0 315 748 behandelt werden. Verbindungen vom Typ h) werden z. B. in der US-A-4 603 101 beschrieben, Verbindungen vom Typ i) z. B. in der US-A-4 491 628 und von J. M. Fréchet et al., J. Imaging Sci. 30, 59-64 (1986).

Es können auch Mischungen der genannten säurespaltbaren Materialien eingesetzt werden. Bevorzugt ist allerdings ein säurespaltbares Material, welches einem der oben genannten Typen zuzuordnen ist. Besonders bevorzugt sind von den Materialien diejenigen, die den Typen a), b), g) und i) angehören. Unter Typ b) sind insbesondere die polymeren Acetale hervorzuheben; von den säurespaltbaren Materialien des Typs g) insbesondere diejenigen, deren Aldehyd- bzw. Ketonkomponente einen Siedepunkt von größer als 150° C, vorzugsweise größer als 200° C, aufweist.

Der Gehalt an säurespaltbarem Material in dem erfindungsgemäßen strahlungsempfindlichen Gemisch sollte bei 1 bis 50 Gew.-%, vorzugsweise bei 5 bis 25 Gew.-%, jeweils bezogen auf das Gesamtgewicht der Schicht, liegen.

Das erfindungsgemäße strahlungsempfindliche Gemisch enthält ferner ein in Wasser unlösliches, in organischem Lösemittel und wäßrigem Alkali aber lösliches, zumindest quellbares Bindemittel. Hierunter zählen vor allem Phenolharze vom Novolak-Typ. Genannt werden Phenol-Formaldehyd-Harze, Kresol-Formaldehyd-Harze, deren Mischkondensate und deren Mischungen.

Daneben können auch noch Vinylpolymerisate wie Polyvinylacetale, Polymethacrylate, Polyacrylate, Polyvinylether, Polyvinylpyrrolidone sowie Styrolpolymerisate, jeweils ggf. durch Comonomere modifiziert, selbst oder in Mischung mit anderen, eingesetzt werden.

Im besonderen sind zu nennen: Polymere von Styrol mit Einheiten von Alkenylsulfonyl-aminocarbonyloxy- oder Cycloalkenylsulfonylaminocarbonyloxy- (EP-A-0 184 804), Polymere der Acryl-, Methacryl-, Malein-, Itaconsäure etc. mit seitenständigen, vernetzenden -CH₂OR-Gruppen (EP-A-0 184 044), Polymerisate aus Vinylmonomeren mit Alkenylphenoleinheiten (EP-A-0 153 682), Polyvinylphenole als Novolakersatz (DE-C-23 22 230), polymere Bindemittel mit seitenständigen, phenolischen Hydroxylgruppen (EP-A-0 212 439 und 0 212 440), Styrol-Maleinsäureanhydrid-Mischpolymerisate (DE-A-31 30 987), Polymere aus ungesättigten (Thio)phosphinsäureiso(thio)cyanaten mit einem aktiven Wasserstoff enthaltenden Polymeren (DE-A-36 15 612 und 36 15 613), Polymere mit Vinylacetat-, Vinylalkohol- und Vinylacetaleinheiten (EP-A-0 216 083) sowie Polyvinylacetale mit Einheiten aus Hydroxyaldehyden (DE-A-36 44 162).

Die Menge des Bindemittels beträgt im allgemeinen 1 bis 90, insbesondere 5 bis 90 Gew.-%, vorzugsweise 50 bis 90 Gew.-%, bezogen auf das Gesamtgewicht der strahlungsempfindlichen Mischung.

Ferner können den erfindungsgemäßen strahlungsempfindlichen Gemischen ggf. Farbstoffe, Pigmente, Weichmacher, Netzmittel und Verlaufmittel, aber auch Polyglykole, Celluloseether, z.B. Ethylcellulose, zur Verbesserung spezieller Erfordernisse wie Flexibilität, Haftung und Glanz zugesetzt werden.

Vorzugsweise wird das erfindungsgemäße strahlungsempfindliche Gemisch in Lösungsmitteln wie Ethylenglykol, Glykolethern wie Glykolmonomethylether, Glykoldimethylether, Glykolmonoethylether oder Propylenglykolmonoalkylethern, insbesondere Propylenglykolmethylether; aliphatische Estern wie Ethylacetat, Hydroxyethylacetat, Alkoxyethylacetat, n-Butylacetat, Propylenglykolmonoalkyletheracetat, insbesondere Propylenglykolmethyletheracetat oder Amylacetat; Ethern wie Dioxan, Ketonen wie Methylethylketon, Methyl-isobutylketon, Cyclopentanon und Cyclohexanon; Dimethylformamid, Dimethylacetamid, Hexamethylphosphorsäureamid, N-Methyl-pyrrolidon, Butyrolacton, Tetrahydrofuran, und in Mischungen derselben gelöst. Besonders bevorzugt werden Glykolether, aliphatische Ester sowie Ketone.

Die mit den Bestandteilen des strahlungsempfindlichen Gemisches entstehenden Lösungen haben in der Regel einen Feststoffgehalt von 5 bis 60 Gew.-%, vorzugsweise bis 50 Gew.-%.

Erfindungsgemäß wird ferner ein strahlungsempfindliches Aufzeichnungsmaterial beansprucht, im wesentlichen bestehend aus einem Substrat und dem darauf aufgetragenen strahlungsempfindlichen Gemisch.

Als Substrate kommen alle Materialien in Frage, aus denen Kondensatoren, Halbleiter, mehrlagige gedruckte Schaltungen oder integrierte Schaltkreise bestehen bzw. hergestellt werden können. Insbesondere sind Oberflächen aus thermisch oxidiertem und/oder mit Aluminium beschichtetem Siliciummaterial zu nennen, die gegebenfalls auch dotiert sein können, einschließlich aller anderen in der Halbleitertechnologie üblichen Substrate wie beispielsweise Siliciumnitrid, Galliumarsenid, Indiumphosphid. Weiterhin kommen in Frage die aus der Flüssigkristalldisplay-Herstellung bekannten Substrate wie Glas, Indium-Zinnoxid; ferner Metallplatten und -folien, beispielsweise aus Aluminium, Kupfer, Zink; Bimetall-und Trimetallfolien, aber auch elektrisch nicht leitende Folien, die mit Metallen bedampft sind, ggf. mit Aluminium beschichtete SiO₂-Materialien und Papier. Diese Substrate können einer Temperatur-Vorbehandlung unterzogen werden, oberflächlich angerauht, angeätzt oder zur Verbesserung erwünschter Eigenschaften, z. B. der Erhöhung der Hydrophilie, mit Chemikalien behandelt sein.

In einer besonderen Ausführungsform kann das strahlungsempfindliche Gemisch zur besseren Haftung in dem Resist oder zwischen dem Resist und dem Substrat einen Haftvermittler enthalten. Bei Silicium- bzw. Siliciumdioxid-Substraten kommen hierfür Haftvermittler vom Aminosilan-Typ, wie z.B. 3-Aminopropyl-triethoxysilan oder Hexamethyl-disilazan in Frage.

Beispiele für Träger, die zur Herstellung von photomechanischen Aufzeichnungsschichten wie Druckformen für den Hochdruck, den Flachdruck, den Siebdruck, den Tiefdruck sowie von Reliefkopien Verwendung finden können, sind Aluminiumplatten, ggf. anodisch oxidiert, gekörnte und/oder silikatisierte Aluminiumplatten, Zinkplatten, Stahlplatten, die ggf. verchromt wurden, sowie Kunststoffolien oder Papier.

Bestrahlt wird das erfindungsgemäße Aufzeichnungsmaterial bildmäßig mit hochenergetischen Strahlungsquellen; bevorzugt ist Elektronen- oder Röntgenstrahlung.

Die Schichtstärke variiert in Abhängigkeit von ihrem Einsatzgebiet. Sie beträgt zwischen 0,1 und 100 µm, insbesondere zwischen 1 und 10 µm.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuderbeschichten und Tauchbeschichten erfolgen. Danach wird das Lösungsmittel durch Verdampfen entfernt, so daß auf der Oberfläche des Substrates die strahlungsempfindliche Schicht zurückbleibt. Die Entfernung des Lösemittels kann gegebenfalls durch Erhitzen der Schicht auf Temperaturen von bis zu 150° C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien ausgewiesene Materialien Anwendung finden. Anschließend wird die Schicht bildmäßig bestrahlt. Besonders bevorzugt sind energiereiche Strahlungen wie Röntgen- oder Elektronenstrahlung. Besonders bevorzugt wird energiereiche Synchrotronstrahlung mit Dosiswerten von 20 bis 200 mJ/cm oder Strahlung eines Elektronenstrahlschreibers. In der strahlungsempfindlichen Schicht wird anschließend durch Entwicklung ein Bildmuster freigelegt, indem die Schicht mit einer Entwicklerlösung behandelt wird, die die bestrahlten Bereiche des Materials löst bzw. entfernt.

Als Entwickler werden Lösungen von alkalischen Reagenzien wie z.B. Silikaten, Metasilikaten, Hydroxiden, Hydrogen- bzw. Dihydrogenphosphaten, Carbonaten bzw. Hydrogencarbonaten, insbesondere von Alkali- oder Ammoniumionen, aber auch Ammoniak und organische Ammoniumbasen und dergleichen verwendet. Der Gehalt dieser Substanzen in der Entwicklerlösung beträgt im allgemeinen 0,1 bis 15 Gew.-%, vorzugsweise 0,5 bis 5 Gew.-%, bezogen auf das Gewicht der Entwicklerlösung.

Zur Erhöhung der Resistenz gegen mechanische und chemische Einflüsse, insbesondere gegen Ätzmedien, können die entwickelten Schichten noch einige Zeit, z.B. 5 bis 40 Minuten, auf erhöhte Temperatur, z.B. oberhalb 100° C, erhitzt werden, wobei diese Wirkung noch durch Belichten mit UV-Strahlung unterstützt werden kann.

Anhand der folgenden Beispiele soll die Herstellung der im erfindungsgemäßen strahlungsempfindlichen Gemisch enthaltenen und zum Teil neuen, aromatisches Chlor oder Brom enthaltenden Verbindungen verdeutlicht werden:

### Herstellungsbeispiel 1: Hexafluortetrabrom-Bisphenol A

0,2 mol Hexafluor-Bisphenol A (2,2-Bis-(4-hydroxy-phenyl)1,1,1,3,3,3-hexafluorpropan) wurden in 100 ml Eisessig unter Rühren gelöst. Bei einer Temperatur von 30 bis 60° C wurde eine Lösung aus 0,8 mol Brom in 100 ml Eisessig zugetropft und das Reaktionsgemisch anschließend so lange unter Rückfluß erhitzt, bis die HBr-Abspaltung beendet war. Die heiße Reaktionsmischung wurde in 2 1 warmes Wasser eingerührt, auf Raumtemperatur abgekühlt, und die entstandenen Kristalle wurden über eine Nutsche abgesaugt. Der Schmelzpunkt des durch Sublimation gereinigten Produktes wurde zu 255° C bestimmt.

### Herstellungsbeispiel 2: 1,1,1-Tris-(3,5-dibrom-4-hydroxy-phenyl)ethan

102 g Tris-(4-hydroxyphenyl)ethan wurden in einer Mischung aus 500 ml Eisessig und 250 ml Wasser suspendiert. Bei 10° C wurden anschließend in die gerührte Suspension 334 g Brom zugetropft. Nach erfolgter Abreaktion des Broms wurden 750 ml Wasser zugegeben und die ausgefallenen Kristalle über eine Nutsche abgesaugt. Sie wurden mit Wasser gewaschen, aus Toluol umkristallisiert und anschließend getrocknet. Das Produkt hatte einen Schmelzpunkt von 276-278° C. Das NMR-Spektrum (CDCl₃) zeigte folgende Signale: CH₃: 2,05 ppm (s, 3H); OH: 5,89 ppm (s, 3H); Phenyl: 7,1 (s, 6H).

### Herstellungsbeispiel 3: N-(2,4,6-Tribromphenyl)-N´-(p-toluolsulfonyl)harnstoff

50 mmol 2,4,6-Tribromanilin wurden in 20 ml Tetrahydrofuran gelöst. Bei Raumtemperatur wurde eine Lösung aus 50 mmol Toluolsulfonylisocyanat in 20 ml Tetrahydrofuran zugetropft und anschließend die gerührte Reaktionsmischung 3 h auf 40° C gehalten. Die ausgefallenen Kristalle wurden über eine Nutsche abgesaugt. Das Produkt hatte nach dem Umkristallisieren aus Toluol einen Schmelzpunkt von 230° C.

### Herstellungsbeispiel 4: Oligomeres aus Tetrabrom-bisphenol A und 1,4-Divinyloxy-butan

56,6 g Tetrabrom-bisphenol A werden in 50 ml Tetrahydrofuran (THF) gelöst und 14,8 g Divinyloxybutan werden zugegeben. Nach 3 h Rühren bei Raumtemperatur werden die leichtflüchtigen Bestandteile am Rotationsverdampfer unter Wasserstrahlvakuum abdestilliert. Der verbleibende farblose Feststoff hat einen Schmelzpunkt von 79° C und zeigt ¹H-NMR-Signale bei 7,25 ppm (s, arom.), 5,60 ppm (q, Acetal) und 1,57 ppm (Methyl).

### Herstellungsbeispiel 5: Tetrabrom-bisphenol-A-bis-(1-ethoxy-ethylether)

56,6 g Tetrabrom-bisphenol A werden in 50 ml Tetrahydrofuran gelöst, dann werden 15 g Vinylethylether und 0,2 g Amberlyst zugegeben. Nach 3 h unter Rückfluß werden 5 g Kaliumcarbonat bei Raumtemperatur zugegeben, es wird eine Stunde nachgerührt, dann filtriert und eingeengt. Das zurückbleibende hochviskose braune Öl zeigt NMR-Signale bei 7,27 ppm (s, arom.), 5,60 ppm (q, Acetal) und 1,60 ppm (Methyl).

### Herstellungsbeispiel 6: Tetrabrom-bisphenol-A-bis-(trimethylsilylether)

56,6 g Tetrabrom-bisphenol A werden in 50 ml THF gelöst. Nach Zugabe von 40 g Pyridin werden 22,5 g Trimethylchlorsilan bei Raumtemperatur zugetropft. Nach einstündigem Erhitzen unter Rückfluß wird durch Ausschütteln mit Diethylether und Wasser aufgearbeitet. Nach dem Einengen der Etherfraktion erhält man Kristalle, die aus Isopropanol umkristallisiert einen Schmelzpunkt von 141° C aufweisen. NMR: 7,26 ppm (s, arom.), 1,60 ppm (s, Me₃Si).

### Herstellungsbeispiel 7: Tetrabrom-bisphenol-A-bis-tetra-hydropyranylether

56,6 g Tetrabrom-bisphenol A werden in 50 ml THF gelöst. Nach Zugabe von 17,5 g Dihydropyran und 0,25 g Amberlyst wird 3 h unter Rückfluß erhitzt, nach dem Abkühlen durch Zugabe von 5 g Kaliumcarbonat neutralisiert, eine Stunde nachgerührt und nach Filtration zur Trockne eingeengt: Öl. NMR: 7,27 ppm (arom.), 6,0 ppm (t, Acetal), 1.57 ppm (Methyl).

### Herstellungsbeispiel 8: Tetrabrom-bisphenol-A-bis(tert,butylcarbonat)

13,6 g Tetrabrom-bisphenol A werden in 50 ml THF gelöst. Nach langsamer Zugabe von 2,65 g Natriumhydrid (50%ig) werden 10,9 g Di-t-butylcarbonat zugetropft. Nach vierstündigem Rühren bei 60° C wird auf Eiswasser gegossen, dreimal mit je 150 ml Ethylacetat ausgeschüttelt, die organische Phase getrocknet und filtriert. Die nach dem Einengen erhaltenen Kristalle ergeben nach Umkristallisation aus Isopropanol einen Schmelzpunkt von 172° C. NMR: 7,22 ppm (arom.), 1,57 ppm (s, 6H), 1,52 ppm (s, 18H).

### Herstellungsbeispiel 9: Tetrabrom-bisphenol-A-diacetat

27,2 g Tetrabrom-bisphenol A werden in 176,6 ml Pyridin gelöst. Dazu werden langsam unter Kühlung 7,8 g Acetylchlorid getropft und das so erhaltene Reaktionsgemisch über Nacht bei Raumtemperatur gehalten. Nach Filtration wird auf Eiswasser gegeben, mit konz. HCl angesäuert. Der ausgefallene Ester wird abgesaugt, in Methylenchlorid aufgenommen und mit Natriumbicarbonatlösung und Wasser gewaschen, getrocknet und eingeengt. Das Produkt wird aus Acetonitril umkristallisiert, F.: 169° C, NMR: 7,42 ppm, 2,40 ppm, 1,68 ppm.

### Herstellungsbeispiel 10: Tetrabrom-bisphenol-A-dibenzoat

Herstellung analog Herstellungsbeispiel 9, jedoch unter Verwendung von Benzoylchlorid anstelle des Acetylchlorids. Weiße Kristalle (aus Acetonitril): F 219° C, NMR 1,75 ppm (6H, Methyl), 14 H arom.

### Herstellungsbeispiel 11: Tetrabrom-bisphenol-A-dibenzolsulfonat

Es wird vorgegangen wie in Herstellungsbeispiel 9, jedoch unter Verwendung von Benzolsulfonylchlorid anstelle von Acetylchlorid. Kristalle (aus Acetonitril), F: 208° C, NMR: 1,65 (6H, Methyl), arom: 14 H.

### Herstellungsbeispiel 12: 1,1,1-Tris-(4-hydroxy-3,5-di-bromphenyl)-ethan-tris-(1-ethoxy-ethylether)

Es wird vorgegangen wie in Herstellungsbeispiel 5 mit dem Unterschied, daß anstelle von Bisphenol A die Verbindung aus Herstellungsbeispiel 2 und der Vinylethylether in dreifachem molarem Überschuß eingesetzt wird. NMR: 1,17 ppm (t, Methyl), 1,57 ppm (d, Methyl), 5,57 ppm (q, Acetal), 7,10 ppm (s, arom.).

### Herstellungsbeispiel 13: Tetrabrom-bisphenol-A-dimethylether

54,4 g Tetrabrom-bisphenol A werden in 200 ml 5%iger NaOH gelöst. Bei Raumtemperatur werden 30,3 g Dimethylsulfat zugetropft, eine Stunde bei Raumtemperatur gerührt, dann auf 40° C erwärmt und 50 ml konz. Ammoniaklösung zugegeben. Das Produkt wird mit Methylenchlorid extrahiert, die organische Phase gewaschen, getrocknet und eingeengt. NMR: 7,3 ppm (s, arom. 4H), 3,88 ppm (s, CH₃O, 6H), 1,6 ppm (s, CH₃, 6H).

Die folgenden Anwendungsbeispiele, in denen Gt Gewichtsteile bedeutet, sollen die Erfindung näher erläutern.

### Beispiel 1

Es wurde eine Beschichtungslösung hergestellt aus
- 17 Gt: eines Kresol-Formaldehyd-Novolaks mit einem Erweichungsbereich von 105-120° C,
- 5 Gt: eines polymeren Acetals aus Benzaldehyd und Hexan1,6-diol analog dem Herstellungsbeispiel 19 in DE-C 27 18 254 und
- 4 Gt: Tetrabrom-Bisphenol A in
- 74 Gt: Propylenglykolmethyletheracetat.

Die Lösung wurde auf einen mit einem Haftvermittler (Hexamethyl-disilazan) behandelten Siliciumwafer bei 3.000 U/min aufgeschleudert. Nach der Trocknung bei 85° C für 30 min in einem Umluftofen wurde eine Schichtdicke von 1 µm erhalten. Bildmäßig bestrahlt wurde mit Synchrotron-Strahlung (BESSY, Berlin, Luftspalt 2 mm) einer Dosis von 44 mJ/cm durch eine Gold-auf-Silicium-Maske. Den experimentellen Aufbau findet man bei A. Heuberger, "X-Ray Lithography", Microelectronic Engineering 3, 535-556 (1985). Nach dem Belichten wurde die Resistschicht 30 Minuten bei Raumtemperatur belassen. Entwickelt wurde mit einem alkalischen Entwickler folgender Zusammensetzung:
5,3 Gt Natriummetasilikat × 9 H₂O,
3,4 Gt Trinatriumphosphat × 12 H₂O,
0,3 Gt Natriumdihydrogenphosphat und
91 Gt vollentsalztes Wasser.

Nach einer Entwicklungsdauer von 30 s erhielt man ein fehlerfreies Bild mit allen Details der Maske. Die Resistflanken waren nicht negativ unterhöhlt und zeigten Winkel von nahezu 90° (in einer Aufnahme eines Rasterelektronenmikroskops (REM)).

### Beispiel 2

Es wurde eine Beschichtungslösung und ein Resist entsprechend Beispiel 1 hergestellt; anstelle des dort angegebenen Starters wurde allerdings Hexafluortetrabrom-Bisphenol A (Herstellungsbeispiel 1) in gleicher Menge zugegeben. Die Ergebnisse hinsichtlich Auflösung und Winkel der Resistflanken zum Trägermaterial nach dem Entwickeln mit dem Entwickler aus Beispiel 1 waren mit denen aus Beispiel 1 vergleichbar.

### Beispiel 3

Es wurde eine Beschichtungslösung entsprechend Beispiel 1 hergestellt. Anstelle von Tetrabrom-Bisphenol A wurde als Starter allerdings Bis(3,5-dibrom-4-hydroxyphenyl)sulfon in Cyclohexanon als Lösungsmittel eingesetzt. Nach Belichtung des Resists wurde dieser in dem Entwickler gemäß Beispiel 1 entwickelt, wobei sich die Entwicklungsdauer auf 20 s verkürzte; die Ergebnisse hinsichtlich Auflösung und Resistflanken entsprachen wiederum denen, die auch in Beispiel 1 entwickelt werden konnten.

### Beispiel 4

Anstelle des in Beispiel 1 eingesetzten Tetrabrom-Bisphenols A wurde Tetrachlor-Bisphenol A in einer Beschichtungslösung, die sonst völlig der des Beispiels 1 entsprach, verwendet. Die Lösung wurde auf einen mit einem Haftvermittler gemäß Beispiel 1 versehenen Siliciumwafer aufgeschleudert (3.000 U/min). Nach 1 Minute Trocknen bei 110° C auf einer beheizten Platte (hotplate) konnte eine Schichtdicke von 1 µm erhalten werden. Bestrahlt wurde mit Synchrotronstrahlung (Dosis: 22 mJ/cm). Die Entwicklung erfolgte nach einer Wartezeit von nur zwei Minuten mit einem Entwickler, der durch Verdünnen des Entwicklers von Beispiel 1 mit entionisiertem Wasser im Verhältnis 1:1 erhalten worden war, innerhalb von 25 Sekunden. Unter diesen Bedingungen war es möglich, Strukturwiedergaben wie in Beispiel 1 zu erzielen.

### Beispiel 5 (Vergleichsbeispiel)

Es wurde eine Beschichtungslösung wie in Beispiel 1 hergestellt, nur daß anstelle des Tetrabrom-Bisphenols A 4-Iodphenol eingesetzt wurde. Beschichtet wurde entsprechend Beispiel 1. In diesem Beispiel konnte allerdings auch mit Synchrotronstrahlung einer Dosis von 250 mJ/cm nach dem Entwickeln gemäß Beispiel 1 kein Bild erhalten werden.

### Beispiel 6

Dieses Beispiel entsprach Beispiel 1, mit der Ausnahme, daß anstelle des dort angegebenen Starters 1,1,1-Tris(3,5-dibrom-4-hydroxyphenyl)ethan(3,5-dibrom-4-hydroxyphenyl)ethan eingesetzt wurde. Bestrahlt wurde bildmäßig mit Synchrotronstrahlung einer Dosis von 30 mJ/cm. Nach dem Entwickeln gemäß Beispiel 1 wurden Ergebnisse in Auflösung und Winkel der Resistflanken zum Träger erhalten, die denen aus Beispiel 1 entsprachen. Zusätzlich wies der Resist gemäß diesem Beispiel einen verbesserten Wärmestand auf.

### Beispiel 7

Anstelle von Tetrabrom-Bisphenol A wurde in diesem Beispiel als Starter 2,4,6-Tribromacetanilid verwendet. Alle übrigen Parameter entsprachen denen des Beispiels 1, mit der Ausnahme, daß mit Synchrotronstrahlung einer Dosis von 170 mJ/cm bildmäßig bestrahlt wurde. Die Ergebnisse waren mit denen aus Beispiel 1 vergleichbar.

### Beispiel 8

In diesem Beispiel wurde als Starter N-(2,4,6-Tribromphenyl)-N'-(p-toluolsulfonyl)harnstoff entsprechend den Vorschriften aus Beispiel 1 eingesetzt. Bestrahlt wurde mit Synchrotronstrahlung einer Dosis von 160 mJ/cm. Alle übrigen Parameter entsprachen denen des Beispiels 1. Die Ergebnisse hinsichtlich Auflösung und Resistflanken waren ebenfalls mit denen des Beispiels 1 vergleichbar.

### Beispiel 9

Anstelle des in Beispiel 1 verwendeten Starters wurde in diesem Beispiel 2,4,6-Tribromphenoxyessigsäure eingesetzt. Bestrahlt wurde mit Synchrotronstrahlung einer Dosis von 160 mJ/cm. Entwickelt wurde mit dem Entwickler aus Beispiel 1, in diesem Falle wurde allerdings mit vollentsalztem Wasser im Verhältnis 1:1 verdünnt. Alle übrigen Parameter waren mit denen aus Beispiel 1 identisch. Es konnte ein fehlerfreies Bild hoher Auflösung gemäß Beispiel 1 erreicht werden.

### Beispiel 10

Dieses Beispiel entsprach Beispiel 9, mit der Ausnahme, daß 3,5-Dibrom-4-hydroxy-benzoesäure als Starter verwendet wurde. Die Ergebnise entsprachen denen aus Beispiel 8.

### Beispiel 11

Es wurde eine Beschichtungslösung hergestellt, enthaltend
- 11 Gt: eines bromierten Poly-(p-hydroxy)styrols mit einem Bromgehalt von 50 Gew.-% (kommerzielles Produkt der Maruzen),
- 11 Gt: des Phenolharzes aus Beispiel 1,
- 5 Gt: eines Acetals aus Benzaldehyd und Phenoxyethanol gemäß der unten angefügten Herstellungsvorschrift in
- 73 Gt: Propylenglykolmethylether-acetat.

Es wurde entsprechend Beispiel 1 beschichtet, bestrahlt und entwickelt. Mit Synchrotronstrahlung einer Dosis von 35 mJ/cm ergaben sich ein Auflösungsvermögen und ein Verhalten der Resistflanken, die mit denen aus Beispiel 1 identisch waren.

### Herstellung von Benzaldehyd-diphenoxyethylacetal

Zu einer Mischung aus 1 mol Benzaldehyd, 2 mol Phenoxyethanol und 1,1 mol Orthoameisensäuretrimethylester wurden unter Kühlung 0,5 g p-Toluolsulfonsäure gegeben. Es wurde 2 h bei Raumtemperatur gerührt. Anschließend wurde an die Mischungsapparatur ein Wasserstrahlvakuum angelegt und jeweils 2 h bei 20° C, 40° C und 60° C weitergerührt. Abschließend wurde die Reaktionsmischung bei 80° C und einem Druck von 0,1 Torr über einen Dünnschichtverdampfer gegeben. Gegebenenfalls kann eine weitere Destillation im Dünnschichtverdampfer bei entsprechend erhöhter Temperatur erfolgen.

Das resultierende Acetal wurde mit Na₂CO₃, K₂CO₃ oder basischem Aluminiumoxid verrührt, wodurch der saure Katalysator neutralisiert wurde. Die festen Bestandteile wurden abgetrennt und das Filtrat im Vakuum eingeengt. Das Acetal konnte in der nun vorliegenden Form in einem Aufzeichnungsmaterial eingesetzt werden. Es wies einen Schmelzpunkt von 22-24° C auf; im IR-Spektrum war kein CO-Signal erkennbar; das NMR-Spektrum (CDCl₃) zeigte ein Acetalsignal bei δ= 5,76 ppm.

### Beispiel 12

Es wurde eine Beschichtungslösung hergestellt, enthaltend
- 22 Gt: eines bromhaltigen Novolaks, hergestellt aus einer Mischung von 2,6-Dimethylol-4-bromphenol und mKresol (Molverhältnis 1:1, saure Kondensation),
- 5 Gt: des Acetals aus Piperonal und Phenoxyethanol in
- 73 Gt: Propylenglykolmethylether-acetat.

Analog zu Beispiel 1 wurde ein Siliciumwafer mit der Beschichtungslösung beschichtet und getrocknet, anschließend mit Synchrotronstrahlung einer Dosis von 81 mJ/cm bildmäßig bestrahlt und entwickelt.

Die Ergebnisse waren mit denen aus Beispiel 1 vergleichbar.

### Herstellung von Piperonal-bis-phenoxyethylacetal

Neben der Säure und dem Lösungsmittel aus Herstellungsbeispiel in Beispiel 11 wurden 1 mol Piperonal und 2 mol Phenoxyethanol vorgelegt. Entsprechend den Ausführungen im Beispiel 11 konnte ein Acetal, das als Öl anfiel, isoliert werden. Es zeigte im NMR-Spektrum (CDCl₃) das für Acetale charakteristische Signal bei δ= 5,66 ppm.

### Beispiel 13

Es wurde eine Beschichtungslösung hergestellt, enthaltend
- 11 Gt: eines Kresol-Formaldehyd-Novolaks mit einem Erweichungsbereich von 105-120° C,
- 11 Gt: eines bromierten Poly-(p-hydroxy)styrols mit einem Bromgehalt von 50 Gew.-% entsprechend den Herstellungsbeispielen 1 bis 3 und
- 5 Gt: eines Acetals aus Cyclohexanon und Phenoxyethanol, das gemäß den Herstellungsangaben aus den Beispielen 11 und 12 dargestellt worden war, in
- 73 Gt: Propylenglykolmethylether-acetat.

Es wurde entsprechend Beispiel 1 beschichtet, bestrahlt und entwickelt. Mit Synchrotronstrahlung einer Dosis von 41 mJ/cm konnten Ergebnisse analog zu Beispiel 1 erreicht werden.

### Beispiel 14

Es wurde eine Beschichtungslösung entsprechend Beispiel 13 hergestellt, mit dem Unterschied, daß als säurespaltbares Material α,p-Bis-trimethylsilyloxy-styrol gemäß dem unten angeführten Herstellungsbeispiel eingesetzt wurde. Die Ergebnisse waren analog zu denen des Beispiels 12.

### Herstellung von α,p-Bis-trimethylsilyloxy-styrol

0,72 mol Trimethylchlorsilan und 1 mol Triethylamin wurden in 100 ml Dimethylformamid vorgelegt. Unter Rühren bei Raumtemperatur wurden 0,35 mol p-Hydroxyacetophenon zugetropft und anschließend 4 h unter Rühren auf l10° C erhitzt. Nach Abkühlen der Reaktionsmischung wurden 100 ml Hexan dazugegeben und mit Wasser gewaschen. Nach der Extraktion wurde die organische Phase einer fraktionierten Destillation unterworfen. Dabei erhielt man 19 g des gewünschten Bisethers (Siedepunkt: 125° C/4 Torr). Das NMR-Spektrum zeigte anhand von enolischen Protonen bei δ = 4,19 und 4,65 ppm, daß die Carbonylgruppen vollständig in Enolethergruppen übergegangen waren.

### Beispiel 15

Es wurde eine Beschichtungslösung hergestellt, enthaltend
- 16 Gt: Poly-(4-tert.-butoxycarbonyl)styrol und
- 4 Gt: Tetrabrom-Bisphenol A in
- 80 Gt: Propylenglykolmethyletheracetat.
Entsprechend Beispiel 1 wurde beschichtet und bestrahlt.

Nach der Bestrahlung wurde 30 min in einem Umluftofen bei 120° C getrocknet. Die abgebildete Probe wurde anschließend wieder entsprechend Beispiel 1 weiterverarbeitet. Die Ergebnisse hinsichtlich Auflösungsvermögen und Resistflankenausgestaltung entsprachen denen aus Beispiel 1.

### Beispiel 16

Es wurde eine Beschichtungslösung hergestellt, enthaltend
- 7 Gt: eines Kresol-Formaldehyd-Novolaks mit einem Erweichungsbereich von 105-120° C,
- 7 Gt: aus bromiertem Poly-(p-hydroxy)styrol mit einem Bromgehalt von 50 Gew.-% entsprechend den Herstellungsbeispielen 1 bis 3 und
- 3 Gt: eines Acetals aus 4-Ethylbenzaldehyd und Phenoxyethanol entsprechend dem unten angeführten Herstellungsbeispiel in
- 83 Gt: Propylenglykolmethyletheracetat.

Entsprechend Beispiel 1 wurde beschichtet. Nach dem Trocknen des Resists in einem Umluftofen gemäß Beispiel 1 wurde eine Trockenschichtdicke von 0,3 µm erhalten.

Bildmäßig bestrahlt wurde mit einem Elektronenstrahlschreiber bei einer Beschleunigungsspannung von 50 kV mit einer Strahlendosis von 1 µC/cm.

Die anschließende Entwicklung mit dem Entwickler gemäß Beispiel 1 konnte bei einer Entwicklungsdauer von 30 s ein fehlerfreies Bild mit einem Auflösungsvermögen von 0,3 µm liefern.

### Herstellung von 4-Ethylbenzaldehyd-bis-phenoxyethyl-acetal

Es wurden 1 mol 4-Ethylbenzaldehyd, 2 mol Phenoxyethanol entsprechend den Herstellungsangaben in Beispiel 11 umgesetzt. Es konnte ein Acetal mit einem Schmelzpunkt von 48° C isoliert werden. Das NMR-Spektrum (CDCl₃) zeigte ein Signal für ein Acetal bei δ = 5,74 ppm.

### Beispiele 17 - 26

Es wurden Beschichtungslösungen mit den Starterverbindungen der Herstellungsbeispiele 4 bis 13 mit folgender Zusammensetzung hergestellt:
- 17 Gt: eines Kresol-Formaldehyd-Novolaks mit einem Erweichungsbereich von 105 - 120° C
- 5 Gt: eines Acetals aus Benzaldehyd und Phenoxyethanol, hergestellt wie in Beispiel 11 beschrieben,
- 4 Gt: des jeweiligen Starters in
- 74 Gt: Propylenglykolmethyletheracetat.

Die Herstellung des Resistfilms sowie die Bestrahlung mit Röntgenstrahlung erfolgte in Analogie zu Beispiel 1. Nach Entwicklung unter den in Tab. 1 angegebenen Bedingungen wurden jeweils Strukturwiedergaben erzielt, die den auf der Maske enthaltenen 0,3 µm Strukturen entsprachen.

Die Resistflanken waren nicht negativ unterhöhlt und wiesen einen Flankenwinkel von nahezu 90° bei REM-Beurteilung auf.

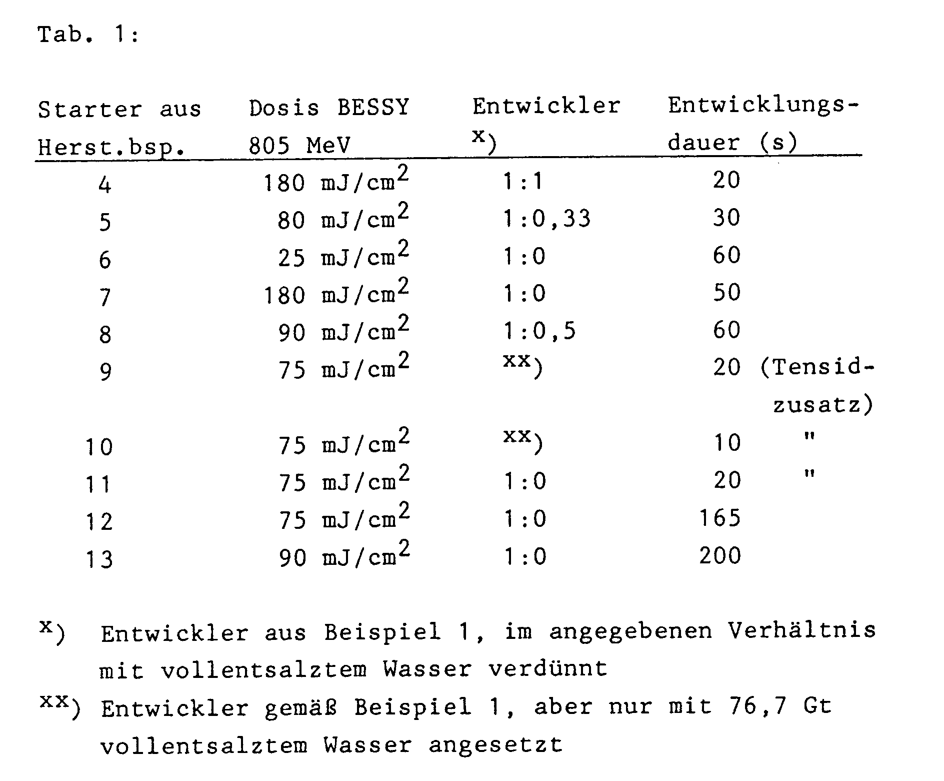

### Beispiel 27:

Es wurde verfahren wie in Beispiel 17, jedoch mit dem Unterschied, daß als Starter die Verbindung aus Herstellungsbeispiel 5 und als Lösungsinhibitor der Silylether gem. Beispiel 14 verwendet wurde. Nach einer bildmäßigen Bestrahlung mit 90 mJ/cm und einer Entwicklung mit 1:0,5 verdünntem Entwickler gemäß Beispiel 1 wurde innerhalb von 60 s ein sehr gutes Resistmuster erhalten.

### Beispiel 28:

Analog erhält man bei Verwendung des Starters aus Herstellungsbeispiel 10 und Orthoameisensäure-tris-phenoxyethylester nach Bestrahlung mit 90 mJ/cm und Entwicklung während 150 s in dem Entwickler von Beispiel 1 ein sehr gutes Resistmuster.

## Patentansprüche

1. Positiv arbeitendes strahlungsempfindliches Gemisch, enthaltend eine Verbindung, die unter Einwirkung von Röntgen- oder Elektronenstrahlung eine Säure bildet, sowie eine Verbindung, die durch Säure spaltbar ist, dadurch gekennzeichnet, daß die säurebildende Verbindung eine Verbindung der allgemeinen Formel I 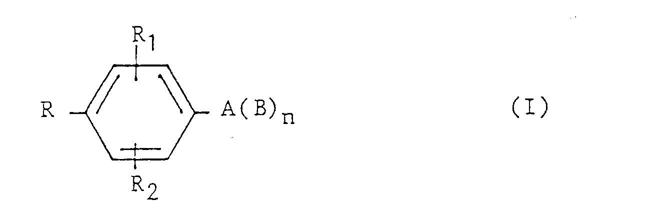 mit mindestens einem aromatisch gebundenen Chlor- oder bromatom ist, wobei
R Carboxyl, OR' oder SR',
R₁ und gleich oder verschieden sind und Wasserstoff,
R₂ Chlor, Brom, Alkyl, ggf. substituiert durch Aryl-, Alkoxy-, Aryloxy-, Hydroxygruppen oder Fluoratome; Aryl, ggf. substituiert durch Alkoxy-, Aryloxy-, Hydroxygruppen oder Halogenatome,
R' Wasserstoff, Alkyl, ggf. substituiert durch Aryl-, Alkoxy-, Aryloxy-, Hydroxygruppen oder Fluoratome; Aryl, ggf. substituiert durch Alkoxy-, Aryloxy-, Hydroxygruppen oder Halogenatome; Acyl, Alkylsulfonyl, Arylsulfonyl, Alkoxycarbonyl, Triorganosilyl, Triorganostannyl oder eine Alkylen-, Arylen-, Bisacyl-, Sulfonyl-, Alkylendisulfonyl-, Arylendisulfonyl-, Diorganosilyl- oder Diorganostannylgruppe ist, deren zweite Valenz an das O-Atom einer weiteren Einheit der Formel I gebunden ist, wobei die Alkylen- und Arylengruppen entsprechend substituiert sein können wie die Alkyl-und Arylreste,
und n 0 bis 3 bedeutet, wobei
für n = 0:
A Wasserstoff, Chlor, Brom, Alkyl, ggf. substituiert durch Alkoxy-, Aryloxy-, Hydroxy-, Arylreste oder Fluoratome; Aryl, ggf. substituiert durch Alkoxy-, Aryloxy-, Hydroxy-, Carboxylreste oder Halogenatome,
für n = 1:
A eine Einfachbindung, -O-, -S-, -SO₂-, -NH-, -NR₃-, Alkylen, Perfluoralkylen,
für n = 2:
A 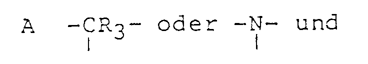 für n = 3: 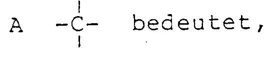 sowie
B 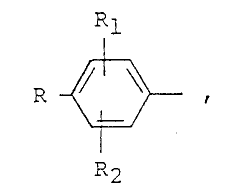 Carboxyl, substituiertes Carbonyl, insbesondere Alkyl- oder Arylcarbonyl, Carboxyalkyl sowie substituiertes Sulfonylimidocarbonyl und
R₃ Alkyl, insbesondere (C₁-C₃)Alkyl, oder Aryl, insbesondere Phenyl, bedeutet, und diese Verbindung
einen pKₐ-Wert von kleiner als 12 aufweist oder ein Derivat einer Verbindung mit einem derartigen pKₐ-Wert ist.

2. Strahlungsempfindliches Gemisch nach Anspruch 1, dadurch gekennzeichnet, daß die säurebildende Verbindung einen pKₐ-Wert von 6 bis 10 aufweist bzw. ein Derivat einer derartigen Verbindung ist.

3. Strahlungsempfindliches Gemisch nach den Ansprüchen 1 oder 2, dadurch gekennzeichnet, daß die säurebildende Verbindung monomer ist und das Gemisch ein in Wasser unlösliches, in wäßrigem Alkali aber lösliches polymeres Bindemittel enthält.

4. Positiv arbeitendes strahlungsempfindliches Aufzeichnungsmaterial für Röntgen- oder Elektronenstrahlung, im wesentlichen bestehend aus einem Träger und einer strahlungsempfindlichen Schicht, dadurch gekennzeichnet, daß ein strahlungsempfindliches Gemisch gemäß den Ansprüchen 1 bis 3 verwendet wird.

5. Verfahren zur Herstellung eines positiven Resistbildes, dadurch gekennzeichnet, daß das Aufzeichnungsmaterial gemäß Anspruch 4 mit Röntgen- oder Elektronenstrahlung bildmäßig bestrahlt und anschließend mit einem wäßrig-alkalischen Entwickler entwickelt wird.

## Claims

1. A positive radiation-sensitive mixture, containing a compound which forms an acid under the action of X-ray or electron radiation, and an acid-cleavable compound, wherein the acid-forming compound is a compound of the formula I 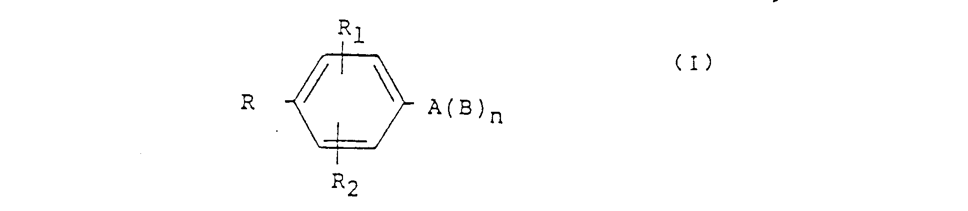 containing at least one aromatically bound chlorine or bromine atom, where
R denotes carboxyl, OR' or SR',
R₁ and R₂ are identical or different and denote hydrogen, chlorine, bromine, alkyl which is optionally substituted by aryl, alkoxy, aryloxy or hydroxyl groups or by fluorine atoms; or aryl which is optionally substituted by alkoxy, aryloxy or hydroxyl groups or by halogen atoms,
R' denotes hydrogen, alkyl which is optionally substituted by aryl, alkoxy, aryloxy or hydroxyl groups or by fluorine atoms; aryl which is optionally substituted by alkoxy, aryloxy or hydroxyl groups or by halogen atoms;
acyl, alkylsulfonyl, arylsulfonyl, alkoxycarbonyl, triorganosilyl, triorganostannyl or is an alkylene, arylene, bisacyl, sulfonyl, alkylenedisulfonyl, arylenedisulfonyl, diorganosilyl or diorganostannyl group whose second valency is bonded to the 0 atom of a further unit of the formula I, it being possible for the alkylene and arylene groups to be substituted in corresponding manner to the alkyl and aryl radicals,
and n denotes 0 to 3, where
for n = 0:
A denotes hydrogen, chlorine, bromine, alkyl which is optionally substituted by alkoxy, aryloxy, hydroxyl or aryl radicals or by fluorine atoms; or aryl which is optionally substituted by alkoxy, aryloxy, hydroxyl or carboxyl radicals or by halogen atoms,
for n = 1:
A denotes a single bond, -O-, -S-, -SO₂-, -NH-, -NR₃-, alkylene or perfluoroalkylene, for n = 2: A denotes 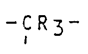 or 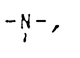 and for n = 3: A denotes 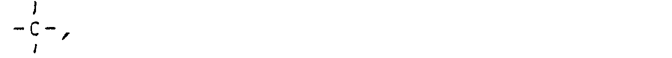 and B denotes 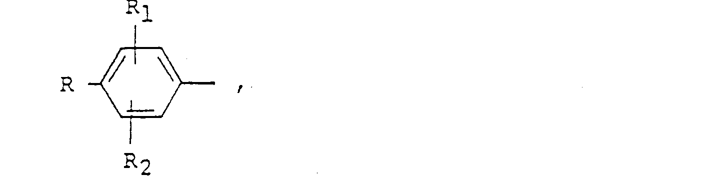 carboxyl, substituted carbonyl, in particular alkyl carbonyl or arylcarbonyl, carboxyalkyl or substituted sulfonylimidocartYonyl, and R₃ denotes alkyl, in particular (C₁-C₃)alkyl, or aryl, in particular phenyl, and this compound has a pKₐ value of less than 12 or is a derivative of a compound having such a pKₐ value.

2. A radiation-sensitive mixture as claimed in claim 1, wherein the acid-forming compound has a pKₐ value of 6 to 10 or is a derivative of such a compound.

3. A radiation-sensitive mixture as claimed in claim 1 or 2, wherein the acid-forming compound is monomeric and the mixture contains a polymeric binder which is insoluble in water, but soluble in aqueous alkali.

4. A positive-working radiation-sensitive recording material for X-ray or electron radiation, consisting essentially of a support and a radiation-sensitive layer, wherein the radiation-sensitive mixture as claimed in any of claims 1 to 3 is used.

5. A process for the production of a positive resist image, wherein the recording material as claimed in claims 4 is imagewise irradiated by X-ray or electron radiation and subsequently developed using an aqueous-alkaline developer.

## Revendications

1. Composition sensible aux radiations, travaillant en positif, contenant un composé qui forme un acide sous l'effet d'un faisceau d'électrons ou de rayons X, ainsi qu'un composé qui est dissociable par un acide, caractérisée en ce que le composé générateur d'acide est un composé de formule générale 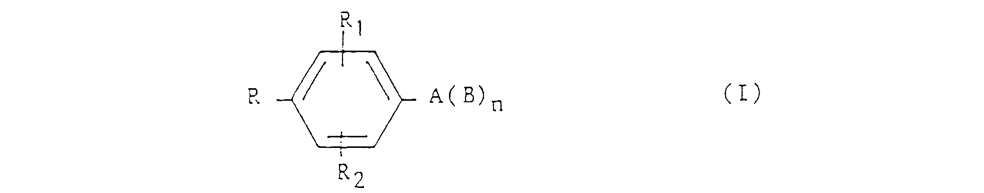 comportant au moins un atome de chlore ou de brome lié au cycle aromatique,
formule dans laquelle
R représente un groupe carboxy, OR' ou SR',
R₁ et R₂ sont identiques ou différents et représentent un atome d'hydrogène, de chlore ou de brome, ou un radical alkyle éventuellement substitué par des atomes de fluor ou par des groupes aryle, alcoxy, aryloxy, hydroxy; un radical aryle éventuellement substitué par des atomes d'halogène ou par des groupes alcoxy, aryloxy, hydroxy,
R' représente un atome d'hydrogène ou un radical alkyle éventuellement substitué par des atomes de fluor ou par des groupes aryle, alcoxy, aryloxy, hydroxy; un radical aryle éventuellement substitué par des atomes d'halogène ou par des groupes alcoxy, aryloxy, hydroxy; un radical acyle, alkylsulfonyle, arylsulfonyle, alcoxycarbonyle, triorganosilyle, triorganostannyle, ou un groupe alkylène, arylène, bis-acyle, sulfonyle, alkylènedisulfonyle, arylènedisulfonyle, diorganosilyle ou diorganostannyle, dont la seconde valence de l'atome d'oxygène est liée à un autre motif de formule I, les groupes alkylène et arylène pouvant être substitués de la même façon que les radicaux alkyle et aryle, et
n va de 0 à 3,
pour n = 0:
A représentant un atome d'hydrogène, de chlore ou de brome, ou un radical alkyle éventuellement substitué par des atomes de fluor ou par des groupes alcoxy, aryloxy, hydroxy, aryle; un radical aryle éventuellement substitué par des atomes d'halogène ou par des groupes alcoxy, aryloxy, hydroxy, carboxy,
pour n = 1:
A représentant une liaison simple, -O-, -S-, -SO₂-, -NH-, -NR₃- ou un groupe alkylène ou perfluoroalkylène, pour n = 2:
A représentant 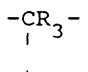 ou 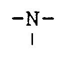 et pour n = 3: A représentant 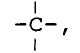 et B représente 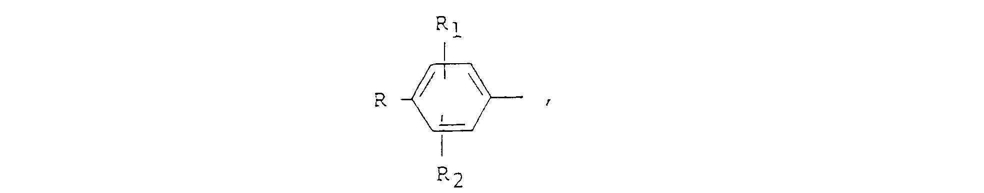 le groupe carboxy, un groupe carbonyle substitué, en particulier alkyl- ou arylcarbonyle, un groupe carboxyalkyle ainsi qu'un groupe sulfonylimidocarbonyle substitué, et
R₃ représente un groupe alkyle, en particulier alkyle en C₁-C₃, ou aryle, en particulier le groupe phényle, et ce composé présente un pKₐ inférieur à 12, ou un dérivé d'un composé ayant un tel pKₐ.

2. Composition sensible aux radiations selon la revendication 1, caractérisée en ce que le composé générateur d'acide présente un pKₐ de 6 à 10, ou est un dérivé d'un tel composé.

3. composition sensible aux radiations selon la revendication 1 ou 2, caractérisée en ce que le composé générateur d'acide est un monomère et la composition contient un liant polymère insoluble dans l'eau, mais soluble dans une solution aqueuse-alcaline.

4. Matériau de reprographie sensible aux radiations, travaillant en positif, pour irradiation par des rayons X ou un faisceau d'électrons, essentiellement constitué d'un support et d'une couche sensible aux radiations, caractérisé en ce que l'on utilise une composition sensible aux radiations selon les revendications 1 à 3.

5. Procédé pour la production d'une image de résist positive, caractérisé en ce que le matériau de reprographie selon la revendication 4 est irradié selon l'image avec des rayons X ou un faisceau d'électrons, et ensuite est développée avec un révélateur aqueux-alcalin.
